# EUROPEAN PATENT SPECIFICATION

(11) **EP 3 414 319 B1**
(45) Date of publication and mention of the grant of the patent: **24.07.2024**
(21) Application number: 17750748.0
(22) Date of filing: 09.02.2017
(51) Int. Cl.: C12N 5/071, C12N 5/02, C12N 5/00, C12M 1/00, C12M 3/00, C12M 1/22, C12M 3/06

(54) **MODEL SYSTEM OF LIVER FIBROSIS AND METHOD OF MAKING AND USING THE SAME**
MODELLSYSTEM FÜR LEBERFIBROSE UND VERFAHREN ZUR HERSTELLUNG UND VERWENDUNG DAVON
SYSTÈME MODÈLE DE FIBROSE HÉPATIQUE ET SON PROCÉDÉ DE FABRICATION ET D'UTILISATION

(30) Priority: 10.02.2016 US 201662293469 P
(43) Date of publication of application: 19.12.2018
(73) Proprietor: Wake Forest University Health Sciences, Winston-Salem, NC 27157 (US)
(72) Inventor: MARINI, Frank C., Winston-Salem North Carolina 27106 (US); SOKER, Shay, Greensboro North Carolina 27410 (US)
(74) Representative: Marks & Clerk LLP
(86) International application number: PCT/US2017/017158
(87) International publication number: WO 2017/139455

(56) References cited:
- WO-A1-2014/151921
- US-A1- 2004 110 289
- US-A1- 2011 014 126
- PEDRO M. BAPTISTA ET AL: "The use of whole organ decellularization for the generation of a vascularized liver organoid", HEPATOLOGY, vol. 53, no. 2, 1 February 2011 (2011-02-01), pages 604 - 617, XP055268730, ISSN: 0270-9139, DOI: 10.1002/hep.24067
- PAUL M. VAN MIDWOUD ET AL: "A microfluidic approach for in vitro assessment of interorgan interactions in drug metabolism using intestinal and liver slices", LAB ON A CHIP, vol. 10, no. 20, 1 January 2010 (2010-01-01), pages 2778, XP055604443, ISSN: 1473-0197, DOI: 10.1039/c0lc00043d
- GIUSEPPE MAZZA ET AL: "Decellularized human liver as a natural 3D-scaffold for liver bioengineering and transplantation", SCIENTIFIC REPORTS, vol. 5, 7 August 2015 (2015-08-07), pages 13079, XP055302497, DOI: 10.1038/srep13079
- BOVENKAMP ET AL.: "Liver fibrosis in vitro: Cell culture models and precision-cut liver slices", TOXICOL IN VITRO, vol. 21, no. 4, 2007, pages 545 - 57, XP022025269
- HANDA ET AL.: "Assembly of Human Organs from Stem Cells to Study Liver Disease", AM J PATHOL., vol. 184, no. 2, 2014, pages 348 - 57, XP055177481
- KAUR ET AL.: "Hepatic Progenitor Cells in Action Liver Regeneration or Fibrosis?", AM J PATHOL., vol. 185, no. 9, 6 August 2015 (2015-08-06), pages 2342 - 50, XP055409070
- AHSAN ET AL.: "Kinetics of liver macrophages (Kupffer cells) in SIV-infected macaques", VIROLOGY, vol. 446, no. 1-2, 2013, pages 77 - 85, XP028727685
- EDMONDSON ET AL.: "Three-Dimensional Cell Culture Systems and Their Applications in Drug Discovery and Cell -Based Biosensors", ASSAY DRUG DEV TECHNOL., vol. 12, no. 4, 2014, pages 207 - 18, XP055409077
- FAULK ET AL.: "Decellularization and Cell Seeding of Whole Liver Biologic Scaffolds Composed of Extracellular Matrix", J CLIN EXP HEPATOL., vol. 5, no. 1, 2015, pages 69 - 80, XP055202246

## Description

### Background of the Invention

Chronic liver injury of various etiologies can cause liver fibrosis, which is characterized by hepatic stellate cell (HSC) activation, proliferation and the progressive accumulation of extracellular matrix in the liver. While acute fibrosis of the liver is typically asymptomatic and reversible, chronic fibrosis can cause permanent damage to the liver, and the only effective treatment to date is a liver transplant.

With no effective treatment for liver fibrosis yet available, research of the mechanisms underlying the development of disease and/or toxicity-induced liver fibrosis is ongoing. The use of cell culture models with cell lines or viable liver slices for such studies have been reported. However, these testing platforms have major limitations of pertinence to real liver tissue and/or a lack of viability.

Baptista et al., (Hepatology 2011, 53(2): 604-617): describe the use of whole organ decellularization for the generation of a vascularized liver organoid.

WO2017139455 (PCT/US2017/017158) describes a model system for liver fibrosis, including a liver extracellular matrix, and a combination of mammalian liver cells (e.g., primary liver cells) on the matrix.

Thus, improved model systems of liver fibrosis are needed, particularly model systems useful for the screening of anti-fibrotic agents.

### Summary of the Invention

The invention is as defined in claims 1-15.

Provided herein are model systems of liver fibrosis useful for screening agents for anti-fibrotic activity, useful for study of the mechanisms of fibrosis in the liver, etc.

Thus, provided herein is a model system for liver fibrosis, said system including a liver extracellular matrix (*e.g.,* a decellularized liver tissue such as a decellularized liver disk), and a combination of mammalian liver cells (*e.g.,* primary liver cells) on said matrix, the combination of liver cells including *(a)* liver progenitor cells, *(b)* Kupffer cells, and *(c)* hepatic stellate cells, and by number, from 70 to 90 percent liver progenitor cells, from 5 to 20 percent Kupffer cells, and from 5 to 20 percent hepatic stellate cells.

In some embodiments, the hepatic stellate cells are activated hepatic stellate cells and/or myofibroblasts (*e.g.,* express EZH2).

In some embodiments, the system is provided in a tissue culture dish. In some embodiments, the system is provided in a modular and/or microfluidic device. In some embodiments, the system is implantable *in vivo.*

Also provided is a method of screening activity of an agent of interest in modulating liver fibrosis, which includes: *(a)* providing a model system as taught herein, *(b)* contacting said agent of interest to said model system, *(c)* measuring fibrosis in the model system, and *(d)* determining whether the fibrosis is increased or decreased in response to the contacting, to thereby screen the activity of the agent of interest in modulating liver fibrosis.

In some embodiments, the measuring comprises measuring the activity of EZH2 in the model system. In some embodiments, the measuring comprises optical clearing (*e.g.,* inCITE optical clearing) and analysis.

In some embodiments, the agent of interest is an EZH2 inhibitor (*e.g.,* GSK-126), an angiotension type 1 (AT1) receptor blocker (*e.g.,* lostatin), halofuginone, a lysyl oxidase or lox-like enzyme inhibitor, an A_{2B} adenosine receptor antagonist, or a monoclinal antibody (*e.g.,* GS-6624 (simtuzumab)).

Further provided is a method of making a model system as taught herein, which may include: *(a)* providing a liver extracellular matrix, *(b)* seeding said liver progenitor cells, Kupffer cells and hepatic stellate cells onto said liver extracellular matrix, and *(c)* growing said cells on said matrix *in vitro,* (d) inducing fibrosis of said cells to thereby form said model system for liver fibrosis, wherein said inducing comprises activating said hepatic stellate cells by administering a pro-fibrogenic cytokine or chemical to said model system.

The present invention is explained in greater detail in the drawings herein and the specification set forth below.

### Brief Description of the Drawings

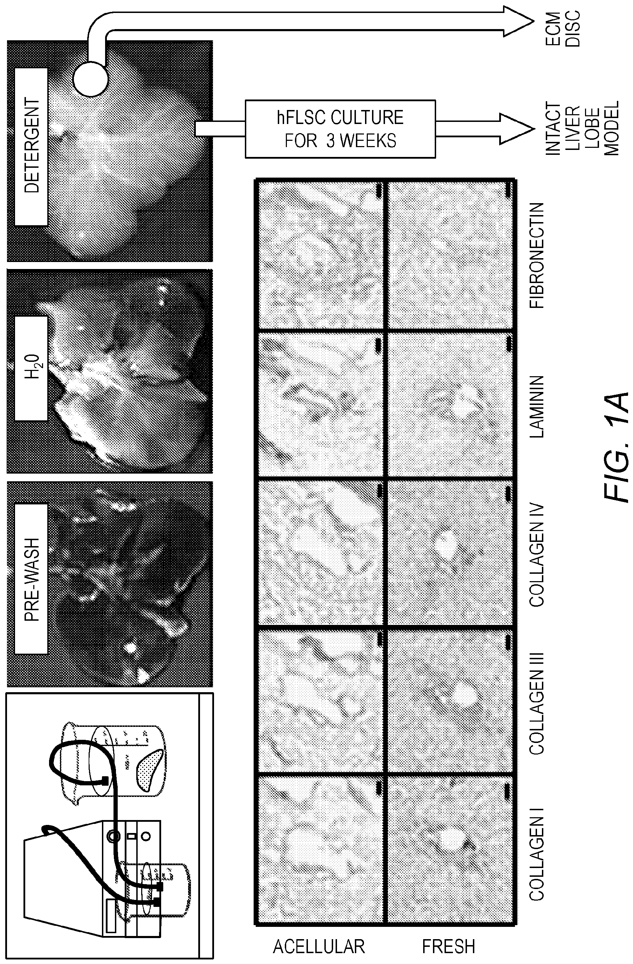

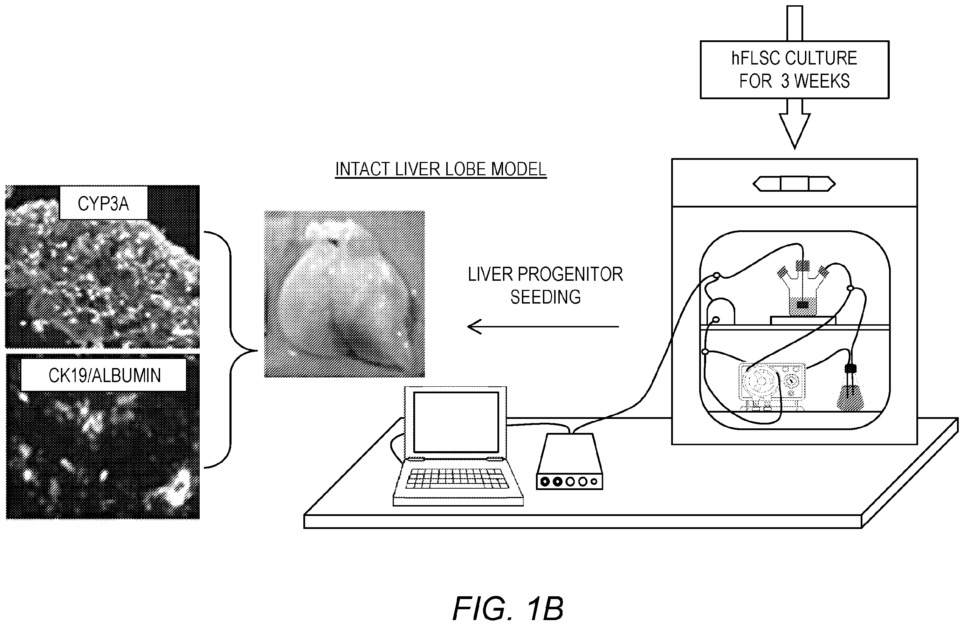

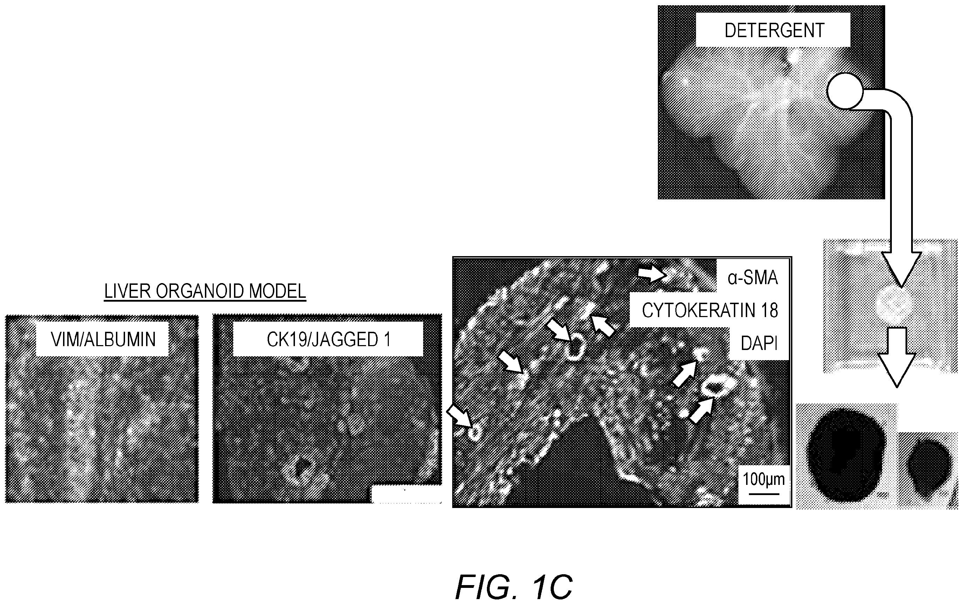

**Fig. 1A****-****Fig. 1C****. Models of bioengineered liver tissue.** **Fig. 1A****:** Liver decellularization process and characterization of the ECM in acellular ferret liver and fresh liver tissue, showing preservation of important liver ECM molecules. **Fig. 1B****:** Intact liver lobe model: Human liver progenitors were infused into an acellular ferret liver ECM using a specialized bioreactor system. After 3 weeks in culture, the liver progenitors differentiated to functional hepatocytes, expressing CYP3A and albumin and CK19⁺ biliary structures. **Fig. 1C****:** Liver organoid model: 8 mm discs "punched" from acellular liver ECM and seeded with human liver progenitors. After 3 weeks, spheroids of 0.5-2 mm in diameter were observed, containing biliary structures (arrows) and hepatocyte clusters (CK18 and albumin - Alb). Abundant stellate cells, expressing Jagged-1, α-SMA and vimentin (Vim) were surrounding the biliary and hepatocytic structures (Bar size=100µm).

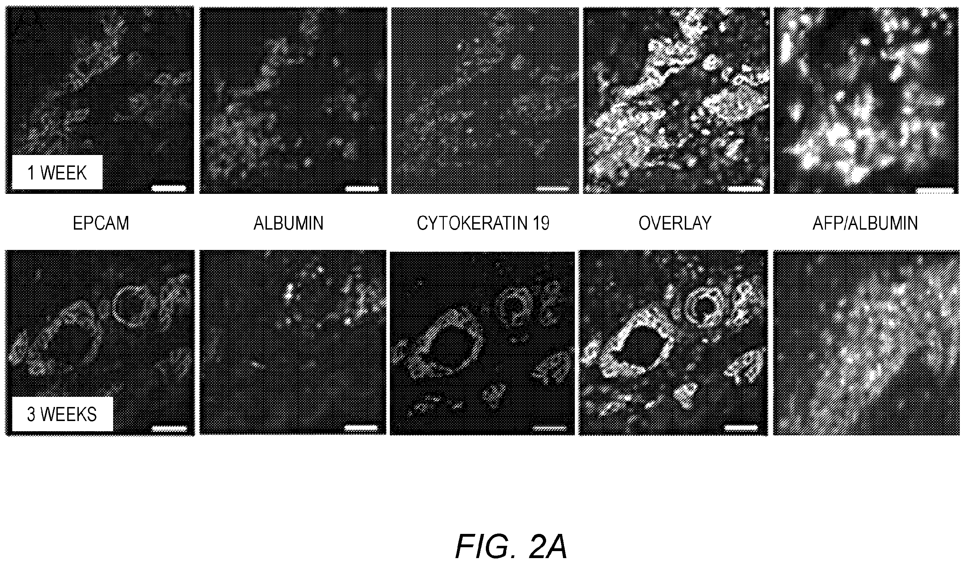

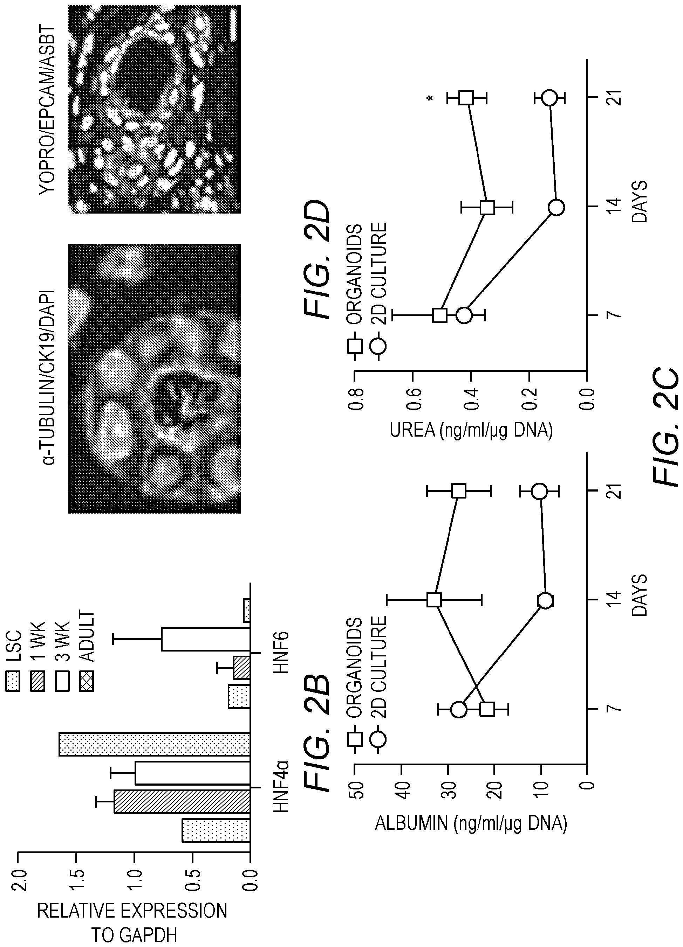

**Fig. 2A****-****Fig. 2C****. Tissue maturation of the liver organoids.** **Fig. 2A****:** Distribution and phenotypic characteristics of LPCs during 1 and 3 weeks of differentiation in culture. Cells were stained for epithelial cell adhesion molecule (EpCAM), albumin (ALB), a-fetoprotein (AFP), cytokeratin19 (CK19) and for cell nuclei (DAPI). **Fig. 2B****:** RT-PCR analysis of the expression of hepatic transcription factors hepatocyte nuclear factor (HNF) 4a, which regulates hepatocytic differentiation, and HNF6, which regulate bile epithelial differentiation, in freshly isolated LPCs, liver organoids after 1 and 3 weeks differentiation, and in adult liver tissue. **Fig. 2C****:** Measurements of albumin secretion and urea concentration in conditioned media of liver organoids and LPCs in culture dishes during 3 weeks of differentiation. **Fig. 2D****:** Characterization of ductular structures for expression of CK19 and acetylated a-tubulin (top) and EpCAM and apical sodium dependent bile transporter (ASBT) (bottom).

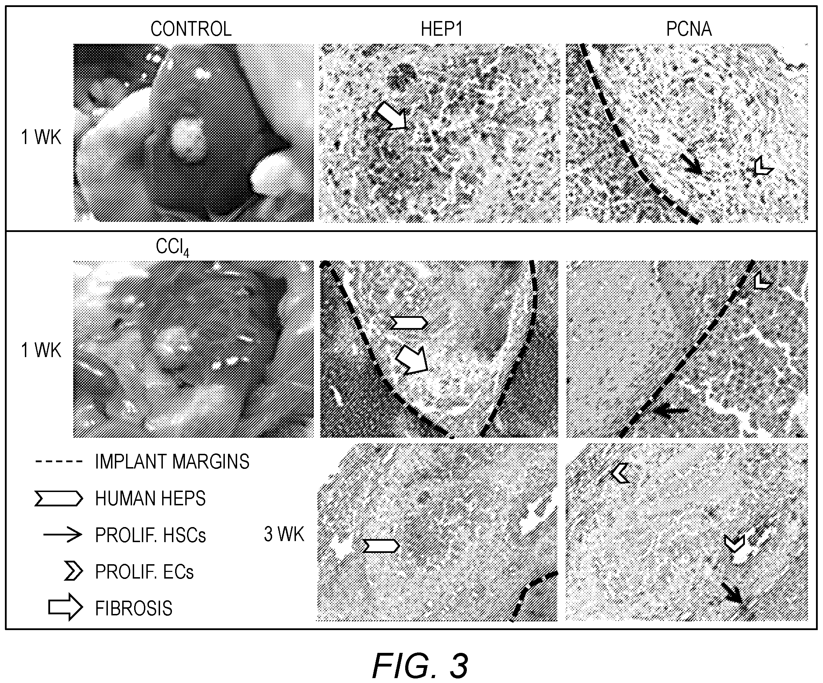

**Fig. 3****. The effect of CCl₄ treatment on implanted liver organoids.** Liver organoids were inserted on top of mouse livers via a small hole carved with a 8mm biopsy punch and immobilized with fibrin glue. Some of the mice were treated with 4µl/g CCl₄, via bi-weekly subcutaneous injections. Liver organoids were harvested after 1 and 3 weeks and immune-stained for human hepatocytes (Hep-1) and proliferating cells (PCNA). Implant margins are drawn.

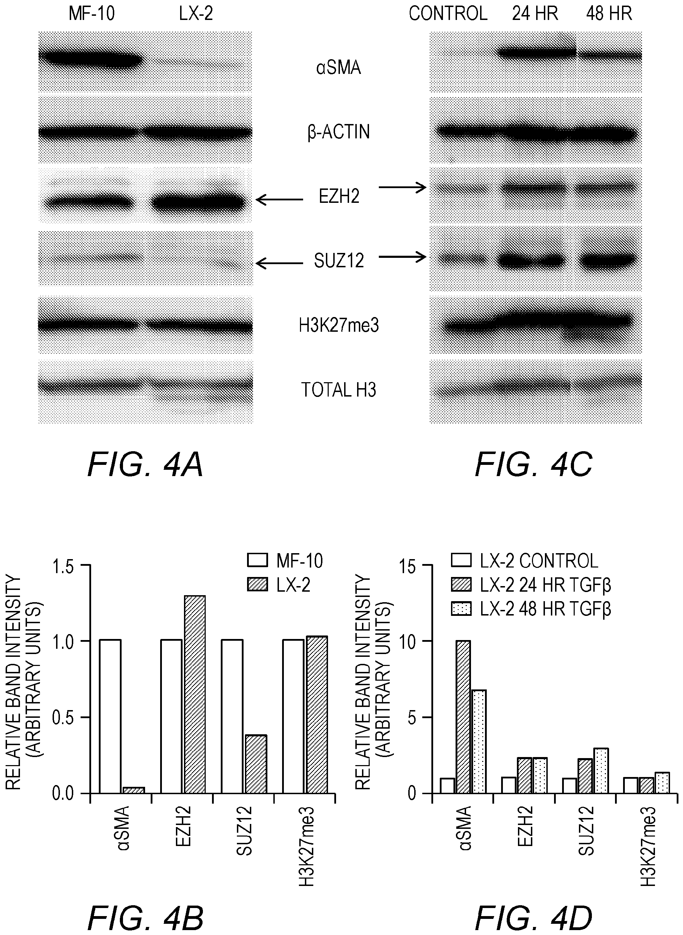

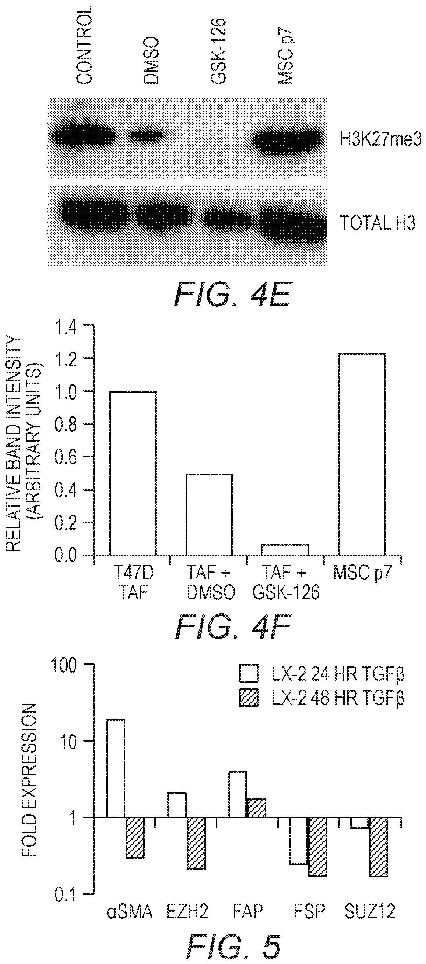

**Fig. 4A****-****Fig. 4F****. Analysis of LX-2 cells.** **Fig. 4A****:** Western blot comparison of αSMA and PRC2 components/markers in Myofibroblasts and LX-2. **Fig. 4B****:** Densitometry analysis of Myofibroblast vs. LX-2 western blot. **Fig. 4C****:** Western blot analysis of αSMA and PRC2 components/markers in LX-2 cells treated with TGFβ for 24 or 48 hours. **Fig. 4D****:** Densitometry analysis of TGFβ treated LX-2. **Fig. 4E****:** Western blot analysis of EZH2 marker (H3K27me3) for EZH2 activity in myofibroblasts transitioned from Mesenchymal Stem Cells treated with GSK-126, a chemical inhibitor of EZH2. DMSO is a vehicle control. **Fig. 4F****:** Densitometry analysis of EZH2 marker demonstrates effective decrease in activity of EZH2 when treated with GSK-126.
**Fig 5****. qPCR Analysis of the effects of TGF-β on LX-2 cells.** Quantitative PCR analysis was performed to probe gene expression of LX-2 cells treated with TGF-β. LX-2 cells (P5) were treated with TGF-β for 24 hr or 48 hrs.

### Detailed Description of Illustrative Embodiments

The present invention is now described more fully hereinafter with reference to the accompanying drawings, in which embodiments of the invention are shown. This invention may, however, be embodied in many different forms and should not be construed as limited to the embodiments set forth herein; rather these embodiments are provided so that this disclosure will be thorough and complete and will fully convey the scope of the invention to those skilled in the art.

The terminology used herein is for the purpose of describing particular embodiments only and is not intended to be limiting of the invention. As used herein, the singular forms "a," "an" and "the" are intended to include plural forms as well, unless the context clearly indicates otherwise. It will be further understood that the terms "comprises" or "comprising," when used in this specification, specify the presence of stated features, integers, steps, operations, elements components and/or groups or combinations thereof, but do not preclude the presence or addition of one or more other features, integers, steps, operations, elements, components and/or groups or combinations thereof.

As used herein, the term "and/or" includes any and all possible combinations or one or more of the associated listed items, as well as the lack of combinations when interpreted in the alternative ("or").

Unless otherwise defined, all terms (including technical and scientific terms) used herein have the same meaning as commonly understood by one of ordinary skill in the art to which this invention belongs. It will be further understood that terms, such as those defined in commonly-used dictionaries, should be interpreted as having a meaning that is consistent with their meaning in the context of the specification and claims and should not be interpreted in an idealized or overly formal sense unless expressly so defined herein. Well-known functions or constructions may not be described in detail for brevity and/or clarity.

"Cells" as used herein are, in general, mammalian cells, such as dog, cat, cow, goat, horse, sheep, mouse, rabbit, rat, ferret, etc. cells. In some preferred embodiments the cells are human cells. Suitable cells are known and commercially available, and/or may be produced in accordance with known techniques. *See, e.g.,* U.S. Patent No. 6,737,270. In some embodiments, cells used in accordance with the present invention are primary cells, taken from tissue and used with no or very few (*e.g.,* 1-3) population doublings, as opposed to those of a cell line (*e.g.,* tumor cells or an artificially immortalized, continuously growing cell population).

"Liver progenitor cells" are known and described, e.g., in U.S. Patent No. 8,709,800, 8,278,105, 9,107,910, 9,334,479 (U.S. 2010/0003752), 10. 456, 425 (U.S. 2011/0129439).

"Kupffer cells" as known in the art are specialized macrophages of the liver that line the walls of the sinusoids.

"Hepatic stellate cells" or "HSCs" are cells found in the perisinusoidal space of the liver. "Activated" hepatic stellate cells as used herein are HSCs having increased levels of expression of EZH2 and/or showing a myofibroblast phenotype. Other markers of the activated HSCs/myofibroblasts in fibrotic livers include, but are not limited to, Fibroblast Activation Protein (FAP), Fibroblast Specific Protein (FSP), α-smooth muscle actin (α-SMA), IL-6, TGF-β, Collagen I, and Vimentin.

Methods of inducing liver fibrosis *in vivo* are known, and include, but are not limited to, administration of carbon tetrachloride (CCl₄), which induces chemical damage to hepatocytes, and bile duct ligation, which involves obstruction of the bile ducts within the liver. Methods of inducing fibrosis *in vitro* may include, but are not limited to, administration of pro-fibrogenic cytokines or chemicals such as CCl₄, methotrexate, allyl alcohol, acetaminophen, transforming growth factor β (TGFβ) dimethylnitrosamine, etc.

Chemotherapy and radiation therapy in the treatment of cancer are two of the most common types of hepatotoxic treatment. Hepatotoxic drugs used to treat cancer include, but are not limited to, adriamycin, methotrexate, 6 mercaptopurine, carboplatin, DTIC (dacarbazine), BiCNU, L-asparaginase, and pentostatin.

Other agents or drugs which may be used to induce liver fibrosis in the model system at taught herein may include, but are not limited to, acebutolol; acetaminophen; actinomycin d; adrenocortical steroids; adriamycin; allopurinol; amoxicillin/clavulanate; anabolic steroids; anti-inflammatory drugs; antithyroid drugs; aspirin; atenolol; azathioprine; captopril; carbamazepine; carbimazole; carmustine; cephalosporins; chlordiazepoxide; chlorpromazine; chlorpromazine/valproic acid; chlorpropamide; chlorpropamide/erythromycin (combination); cimetidine; cloxacillin flecainide; cyclophosphamide; cyclophosphamide/cyclosporine; cyclosporine; dacarbazine; danazol; dantrolene; diazepam; diclofenac; diltiazem; disopyramide; enalapril; enflurane; erythromycin; ethambutol; ethionamide; flurazepam; flutamide; glyburide; gold; griseofulvin; haloperidol; halothane; hydralazine; ibuprofen; imipramine; indomethacin; isoniazid; ketoconazole; labetalol; maprotiline; mercaptopurine; methotrexate; methyldopa; methyltestosterone; metoprolol; mianserin; mitomycin; naproxen; nicotinic acid; nifedipine; nitrofurantoin; nonsteroidal; norethandrolone; oral contraceptives; oxacillin; para-aminosalicylic acid; penicillamine; penicillin; penicillins; phenelzine; phenindione; phenobarbital; phenothiazines; phenylbutazone; phenyloin; phenyloin troleandomycin; piroxicam; probenecid; procainamide; propoxyphene; pyrazinamide; quinidine; quinine; ranitidine; salicylates; sulfonamides; sulindac; tamoxifen; terbinafine HCl (Lamisil, Sporanox); testosterone; tetracyclines; thiabendazole; thioquanine; thorotrast; tolbutamide; tricyclic antidepressants; valproic acid; verapamil; vincristine; and vitamin A. *See* U.S. Patent No. 8,609,671 to Belardinelli et al.

Methods for monitoring or detecting liver fibrosis may include, but are not limited to, histological examination and/or measuring expression of certain markers such as EZH2. Other markers for liver fibrosis that may be measured are provided in U.S. Patent No. 7,972,785 to Hsieh et al.

"EZH2" or "enhancer of zeste homolog 2" is a methyltransferase and component of the polycomb repressor complex (PRC) in activated HSCs. EZH2 is involved in the proliferation of some cancers, and thus EZH2 inhibitors are under study for use in cancer therapies.

Agents of interest in modulating liver fibrosis may include, but are not limited to, EZH2 inhibitors and other inhibitors of chromatin modifying enzymes (e.g., GSK-126, 3-deazaneplanocin A (DZNep), suberoylanilide hydroxamic acid (SAHA), MC1948, MC1945, etc.). Inhibitors of EZH2 are known, and many target the SET domain active site of the protein. *See, e.g.,* WO2011140324 (PCT/US2011/035336), WO2011140325 (PCT/US2011/035340), and WO2012005805 (PCT/US2011/035344).

Other agents of interest may include, but are not limited to, an angiotension type 1 (AT1) receptor blocker (*e.g.,* lostatin); a collagen inhibitor such as halofuginone (*see* U.S. Patent No. 8,668,703); a lysyl oxidase or lox-like enzyme inhibitor; a monoclinal antibody (*e.g.,* GS-6624); an oligopeptide such as that found in U.S. Patent No. 8,957,019 to Lei et al.; a retinoic acid derivative such as that found in US 2010/0113596 to Yang; an A_{2B} adenosine receptor antagonist such as 3-n-propylxanthine (enprofylline), 1,3-dipropyl-8-(p-acrylic) phenylxanthine, or those found in U.S. Patent Nos. 6,825,349 to Kalla et al., 8,609,671 to Belardinelli et al.; a compound such as that found in U.S. Patent No. 7,847,132 to Ishikawa et al., etc.

A "liver extracellular matrix" as used herein means a scaffold containing extracellular matrix proteins normally found in the liver, such as those described in Y. Zhang et al., US 9,938,502 (Patent Application Publication No. US 20130288375). For example, a decellularized liver tissue may be lyophilized and ground into a powder to provide extracellular matrix proteins normally found in the liver, which may then be combined with a biopolymer (e.g., collagen, chitosan, hyaluronic acid, etc.) to form a hydrogel. A liver extracellular matrix may also be provided by the use of a decellularized liver organ or portion thereof (*e.g.,* an individual lobe, or a tissue disk created therefrom). Methods for decelluarization of liver tissue are known and described in US 9, 938, 502 (US 20130288375). *See also* Baptista et al., Hepatology 2011, 53(2): 604-617. The liver extracellular matrix may be from any suitable human or non-human mammal, such as dog, cat, cow, goat, horse, sheep, mouse, rabbit, rat, etc. cells. In some preferred embodiments the liver extracellular matrix is from a ferret.

In some embodiments, the liver extracellular matrix includes one or more proteins selected from collagen I, collagen III, collagen IV, laminin, and fibronectin.

Liver constructs (or "organoids") useful as a model system for liver fibrosis as taught herein may include, in combination: *(a)* liver progenitor cells, *(b)* Kuppfer cells, and/or *(c)* hepatic stellate cells. In general, the cells may be seeded onto liver extracellular matrix (*e.g.,* a decellularized liver or portion thereof) provided *in vitro,* such as in a tissue culture dish (*e.g.,* liver ECM disks in 48-well dish). In some embodiments, the liver progenitor cells may be seeded in an amount by number of from 70 to 90 percent (most preferably about 80 percent, *e.g.,* 3×10⁵); the Kupffer cells may be included in an amount by number of from 5 to 20 percent (most preferably about 10 percent, *e.g.,* 4×10⁴); and/or the hepatic stellate cells may be included in an amount by number of from 5 to 20 percent (most preferably 10 percent, *e.g.,* 4×10⁴).

In some embodiments, the seeded constructs (*e.g.,* in the form of spheroids) are grown *in vitro* to form mature liver structures, e.g., from 1 to 4 weeks, or from 1 to 3 weeks, or from 2 to 3 weeks. Such mature liver structures may include, e.g., biliary ductal structures, clustered hepatoctyes, etc.

***Devices**.* Devices useful for *in vitro* compound screening with the model system of the invention may be produced by (a) providing a substrate or device body (*e.g.,* a tissue culture dish, a microfluidic device, etc.) having at least one chamber formed therein (the chamber preferably having an inlet and outlet opening formed therein); and (b) depositing at least one construct as described above (per se, or as a composition thereof in combination with a hydrogel) in the chamber. The device may be provided in the form of a cartridge for "plug in" or insertion into a larger apparatus including pumps, culture media reservoir(s), detectors, and the like.

The device body may itself be formed of any suitable material or combination of materials. Examples include, but are not limited to, polydimethylsiloxane (PDMS), polystyrene, polymethyl methacrylate (PMMA), polyacrylamide, polyethylene glycol (PEG) including functionalized PEG (*e.g.,* PEG diacrylate, PEG diacrylamide, PEG dimethacrylate, etc., or any of the foregoing PEGs in multi-arm forms, etc.), natural polymers or proteins that can be cross-linked or cured (*e.g*., hyaluronic acid, gelatin, chondroitin sulfate, alginate, etc., including derivatives thereof that are functionalized with chemical groups to support cross linking, and combinations thereof. The device body may be formed by any suitable process, including molding, casting, additive manufacturing (3d printing), lithography, etc., including combinations thereof.

***Storing and shipping of devices.*** Once produced, devices as described above in cartridge form may be used immediately, or prepared for storage and/or transport.

To store and transport the product, a transient protective support media that is a flowable liquid at room temperature (*e.g.,* 25° C), but gels or solidifies at refrigerated temperatures (*e.g.,* 4° C), such as a gelatin mixed with water, may be added into the device to substantially or completely fill the chamber(s), and preferably also any associated conduits. Any inlet and outlet ports are capped with a suitable capping element (*e.g.,* a plug) or capping material (*e.g.,* wax). The device is then packaged together with a cooling element *(e.g.,* ice, dry ice, a thermoelectric chiller, etc.) and all placed in a (preferably insulated) package.

Alternatively, to store and transport the product, a transient protective support media that is a flowable liquid at cooled temperature (*e.g.,* 4° C), but gels or solidifies at warmed temperatures such as room temperature (*e.g.,* 20° C) or body temperature (*e.g.,* 37° C), may be provided, such as poly(N-isopropylacrylamide and poly(ethylene glycol) block copolymers.

Upon receipt, the end user may simply remove the device from the associated package and cooling element, allow the temperature to rise or fall (depending on the choice of transient protective support media), uncaps any ports, and removes the transient protective support media with a syringe (*e.g.,* by flushing with growth media).

***Methods of use of devices.*** Devices described above can be used for *in vitro* screening (including high through-put screening) of an agent of interest (or multiple agents of interest) for pharmacological and/or toxicological activity. Such screening can be carried out by: *(a)* providing a device as described above; *(b)* administering a compound to the construct (*e.g.,* by adding to a growth media being flowed through the chamber containing the construct); and then *(c)* detecting a pharmacological and/or toxicological response to the compound from at least one cell of the construct. Detecting of the response may be carried out by any suitable technique, including microscopy, histology, immunoassay, etc., including combinations thereof, depending on the particular response, or set of responses, being detected. Such response or responses may be cell death (including senescence and apoptosis), cell growth (*e.g.,* benign and metastatic cell growth), absorption, distribution, metabolism, or excretion (ADME) of a compound, or a physiological response (*e.g.,* upregulation or downregulation of production of a compound by the at least on cell), or any other biological response relevant to pharmacological and/or toxicological activity with regard to liver fibrosis.

In some embodiments, the liver model is processed for optical clarity. In some embodiments, the liver model is fixed and processed by removing lipid therefrom by index-matched Clear Imaging for Tissue Evaluation ("turns tissue into glass"). The inCITE optical clearing and analysis technology, in which whole organ(s) (or organoid) can be visualized at a 1µM scale for full cellular level resolution, is described in PCT/US2015/044376, filed August 7, 2015, and published as WO2016023009 on February 11, 2016. The method may be performed, *e.g.,* by contacting a fixed tissue with a composition comprising sodium dodecyl sulfate (SDS), 3-(N,N-Dimethylmyristylammonio)propanesulfonate (SB3-14), Tween^{®} 20 (polysorbate 20), a non-ionic surfactant such as Triton^{™} X-100, sodium deoxycholate, and a salt (*e.g.,* sodium chloride, calcium chloride and/or sodium metaborate). In some embodiments, the composition may comprise phospholipase A2. The tissue may thereafter be contacted with 2'2'-thiodiethanol to prepare for imaging. The cleared tissue, which appears as a "see-thru" or glass-like "jellybean," can then be index matched to microscope objectives and imaged. Each whole mount tissue may require up to 10 days for clearing. Data from this imaging technology may be fully quantitated, and hard metrics for fibrosis (fiber length, width, orientation, amount of fibrosis, anisotropy, etc.) can be assessed and compared to current standard Metvir pathological scoring.

The tissue may be fixed, *e.g.,* by contacting or infusing the tissue with a solution comprising acrylamide and a fixative such as paraformaldehyde, formalin, Zenker's fixative, Helly's fixative, B-5 fixative, Bouin's solution, Hollande's, Gendre's solution, Clarke's solution, Cronoy's solution, Methacarn, Formol acetic alcohol, etc. The solution may also include saponin. The tissue may then be left in contact with the solution (*e.g.,* at 4 degrees Celsius with gentle agitation) for sufficient time to be fixed (*e.g.,* 2, 3, 4 or 5 days).

The present invention is explained in greater detail in the following non-limiting Examples.

### EXAMPLE 1

A bioengineered liver model containing primary liver cells was created on a liver extracellular matrix (decellularized liver disc). Over a 3-week maturation *in vitro,* the bioengineered liver formed small organoids, with native liver anatomy and liver-associated functions.

***In situ* organoid model:** For liver bioengineering, perfusion of detergents through the hepatic circulation yielded an acellular liver scaffold, comprised of native liver ECM and retaining characteristic 3D architecture and shape **(****Fig. 1A****).** Remarkably, the channels of the vascular network appear patent. Onto the non-human liver scaffolds were seeded primary human cells: vascular endothelial cells (EC) to cover the blood vessel channels, and human fetal liver progenitor (LPCs) to reconstitute the parenchyma **(****Fig. 1B****).** Such cell-seeded constructs can be kept in perfusion bioreactors for periods of ≥3 weeks, while the cells organize into tissue structures like that of normal liver, including albumin expressing hepatocyte clusters and CK19-positive biliary ductular structures **(****Fig. 1C****).** Furthermore, these organoids performed common hepatic functions including synthesis of albumin, secretion of urea and metabolism of diazepam to phase I metabolites; temazepam and nordiazepam (generated by CYP2C and CYP3A, respectively), confirming CYP3A staining of the liver organoids **(****Fig. 1B****).**

To simplify and adapt to higher throughput applications, small (8mm diameter, 300µm thick) decellularized liver ECM discs were prepared for seeding LPCs **(****Fig. 1C****).** The LPC repopulated the liver ECM and self-assembled into 3D spheroid structures (organoids), containing hepatocytic and ductular structures similar to that of native liver **(****Fig. 1C****).** Furthermore, progressive cellular organization and differentiation were observed. Large clusters of cells expressing hepatoblast markers (ALB⁺/CK19⁺/EpCAM⁺) and both α-fetoprotein (AFP) and albumin were observed after 1 week in culture, suggesting lineage restriction to hepatoblast **(****Fig. 2A****, Top).** After 3 weeks, there were clear changes in cell phenotype, including ALB⁻/CK19⁺/EpCAM⁺ ductular structures and ALB⁺/CK19⁻/EpCAM⁻clusters, and complete loss of AFP expression, suggesting parallel lineage specification into polarized cholangiocytes and hepatocytes, respectively **(****Fig. 2A****, Bottom).** Gene expression analysis showed expression of HNF4α, a hepatocyte differentiation regulator, and HNF6, a cholangiocyte differentiation major regulator, progressively increased in organoids compared to FLPCs **(****Fig. 2B****).** The liver organoids showed significantly higher albumin and urea secretion compared with LPCs differentiated in culture plates **(****Fig. 2C****)** and the biliary structures showed typical apical-basal polarity, indicated by the presence of primary cilia (stained for α- acetylated tubulin) and a bile salt transporter (ASBT) in the apical membrane **(****Fig. 2D****).**

Altogether, these results indicate that the acellular liver discs provide the proper conditions for LPCs to organize, mature and form functional hepatic organoids, with similar anatomy as the native liver tissue.

**The effect of CCl₄ treatment on implanted liver organoids:** The liver organoids developed *in vitro* and showed both functionality and liver tissue anatomy. Yet, the *in vitro* culture conditions lack multiple factors present *in vivo* including components of the blood and immune cells, to mention a few. Accordingly, we implanted organoids on top the liver of nude mice by creating a small hole with a biopsy punch and immobilized them with fibrin glue. Organoids harvested after 1 week showed many viable human hepatocytes and a large number of multiple proliferating stroma (stellate) and endothelial cells **(****Fig. 3****, top panels).** In parallel, we treated some on the implanted mice with 4ml/g of CCl₄ in olive oil (1:1), via bi-weekly subcutaneous injections. Grossly, organoids harvested after 1 week of CCl₄ treatment did not show marked differences from the control mice. However, a close inspection showed lack of nucleated human hepatocytes within the organoids and early signs of fibrosis. Organoids harvested after 3 weeks of CCl₄ treatment showed a higher number of proliferating stromal and endothelial cells. These results indicate that the liver organoids survived upon implantation and showed signs of fibrosis upon treatment with CCl₄. Neovascularization was also observed within the organoids, probably due to CCl₄-induced injury of the host liver.

In the fibrotic liver tissue, about 90% of myofibroblasts are derived from HSC (Liedtke, C., et al., Experimental liver fibrosis research: update on animal models, legal issues and translational aspects. Fibrogenesis Tissue Repair, 2013. 6(1): p. 19), and EZH2 may be an epigenetic regulator of HSC activation and transition into myofibroblast. It was shown that, like myofibroblasts (MF-10), the HSC cell line (LX-2) expresses EZH2 and the PRC components *in vitro* **(****Fig. 4A, Fig. 4B****).** It was next demonstrated that incubation of LX-2 with TGFβ induced EZH2 activity and PRC machinery, including Suz12, and activity marker H3K27me3 **(****Fig. 4C, Fig. 4D****).**

The EZH2 specific small molecule inhibitor GSK-126 is effective at preventing H3K27me3 in lymphoma and non-small cell lung cancer cell lines *in vitro.* In fact, using GSK-126 to inhibit EZH2 in cancer cell lines that have EZH2 activating mutations resulted in cell death due to reliance on EZH2 in these respective cell lines, whereas it is non-lethal, even at high doses, when the cells do not carry activating EZH2 mutations.

Incubation of tumor-associated fibroblasts (TAF) with GSK-126 resulted in complete loss of H2K27me3 **(****Fig. 4E, Fig. 4F****).**

### EXAMPLE 2

Liver organoids are formed by co-seeding liver progenitor cells (LPC), hepatic stellate cells (HSC) and Kupffer cells (KC). In response to fibrotic inducing conditions, the HSC will become activated, proliferating and initiating a fibrotic process in the organoid. The fibrotic liver organoids will be critically examined via range quantitative measures. *In vitro* and *in vivo* experiments may be performed to determine the role of EZH2 in the transition/activation of HSC to myofibroblasts via assessment of EZH2 expression in HSC (a correlative measure) and by using specific EZH2 inhibition (a direct measure).

Hepatic stellate cells (HSC) are the main driver of liver fibrosis. To date, most experimental models to study HSC *in vitro* use simple, HSC only, 2D culture systems, which poorly represent their role in liver fibrosis *in vivo.* The bioengineered liver organoids taught herein better model and elucidate factors affecting HSC and liver fibrosis.

Fetal liver tissue (Advanced Bioscience Resources, Alameda, CA) is digested, spun at low speed to remove erythrocytes, and plated onto collagen 4 and laminin coated dishes. LPC colonies, appearing after about 10 days, are digested and density centrifugation used to separate parenchymal (LPCs) from non-parenchymal (stellate) cells. Human Kupffer cells (KC) can be purchased from Life Technologies (ThermoFisher Scientific). In order to recapitulate the natural proportions of the different liver cell types, ECM discs, placed inside 48 well dishes, will be seeded with ~80%LPC (3×10⁵), ~10%HSC (4×10⁴) and ~10%KC (4×10⁴). These numbers may be optimized based on the histological results of mature organoids. RPMI medium with 1% fetal bovine serum plus defined supplements (dexamethasone, cAMP, prolactin, glucagon, niacinamide, α-lipoic acid, triiodothyronine, EGF, HDL, HGF, GH) supports LSC growth and differentiation on the 3D liver ECM scaffolds.

The organoids are allowed to mature for 2 weeks because, typically, by this time ductular structures and hepatocyte foci are distinctly visible. Fibrosis will be induced using 3 different modes: 1) Directly, by activation of HSC with 3 known fibrosis-inducing growth factors: TGFb, PDGF-BB and TNFα; 2) Indirectly, by exposing organoids to LPS and IL2, thereby stimulating KC to secrete fibrosis inducing factors; and 3) Inducing liver "injury" using CCl₄ that damages hepatocytes, thereby causing the release fibrosis inducing toxicants. Dose escalating experiments may be performed in order to determine the concentrations that will induce fibrosis without significant cell death.

These organoids can be mass produced for high-throughput testing, and each constituent of the organoid can be manipulated and assessed for its impact on liver fibrosis. The organoids show high levels of expression of EZH2, a methyltransferase and component of the polycomb repressor complex (PRC), in activated HSC, demonstrating the activation of HSCs to the myofibroblast phenotype.

**Immunofluorescence histochemistry:** Fibrotic liver or organoid sections are examined using the inForm software package. Sections will be stained by H&E to demonstrate fibrosis. Fibrotic liver or organoids will also be stained for myofibroblast markers, for example, Collagen I, Desmin, and αSMA. Using the cellSens imaging software, all 3 markers will be multi spectrally imaged to determine colocalization of myofibroblast marker expression within the fibrotic liver. After imaging, inForm will be utilized to determine the percentage of myofibroblasts (as indicated by Collagen I, Desmin, and/or αSMA positive staining). Liver sections will also be analyzed for correlation between EZH2 and myofibroblast presence by colocalization of EZH2/H3K27me3 with myofibroblast markers.

### EXAMPLE 3

HSCs are manipulated in order to control liver fibrosis in the organoids, *in vitro* and *in vivo.* For example, fibrosis may be induced and EZH2 activity may be inhibited with agents known for such activity (*e.g.,* GSK126). Although there is a large proportion of HSC in the organoids, it was found that they do not induce a fibrotic phenotype under the standard liver differentiation/maintenance media. This may be due to the fact that these are primary/quiescent HSCs.

A suite of quantitative imaging methodologies can be used to assign metrics to measure fibrosis in organoids *in vitro* and upon implantation in a pre-clinical model (*e.g.,* mouse liver). Multiple aspects of the fibrotic phenotype may be measured, with primary measures for each of the categories: HSC and KC activation, liver tissue anatomy, function and damage and ECM properties.

The liver organoid model allows rapid screening of anti-fibrotic therapeutic agents, which can be rapidly translated into clinical trials, such as inhibitors of chromatin-modifying enzymes which are currently being tested in human patients.

### References

1. Spaeth, E.L., et al., Mesenchymal stem cell transition to tumor-associated fibroblasts contributes to fibrovascular network expansion and tumor progression. PLoS One, 2009. 4(4): p. e4992.
2. Spaeth, E.L., et al., Mesenchymal CD44 expression contributes to the acquisition of an activated fibroblast phenotype via TWIST activation in the tumor microenvironment. Cancer Res, 2013. 73(17): p. 5347-59.
3. Kidd, S., et al., Origins of the tumor microenvironment: quantitative assessment of adipose-derived and bone marrow-derived stroma. PLoS One, 2012. 7(2): p. e30563.
4. Liedtke, C., et al., Experimental liver fibrosis research: update on animal models, legal issues and translational aspects. Fibrogenesis Tissue Repair, 2013. 6(1): p. 19.
5. Klingberg, F., B. Hinz, and E.S. White, The myofibroblast matrix: implications for tissue repair and fibrosis. J Pathol, 2013. 229(2): p. 298-309.
6. Thijssen, S., et al., Changes in expression of fibrotic markers and histopathological alterations in kidneys of mice chronically exposed to low and high Cd doses. Toxicology, 2007. 238(2-3): p. 200-10.
7. Eming, S.A., P. Martin, and M. Tomic-Canic, Wound repair and regeneration: mechanisms, signaling, and translation. Sci Transl Med, 2014. 6(265): p. 265sr6.
8. Atta, H., et al., Mutant MMP-9 and HGF gene transfer enhance resolution of CCl4-induced liver fibrosis in rats: role of ASH1 and EZH2 methyltransferases repression. PLoS One, 2014. 9(11): p. e112384.
9. Mann, J., et al., MeCP2 controls an epigenetic pathway that promotes myofibroblast transdifferentiation andfibrosis. Gastroenterology, 2010. 138(2): p. 705-14, 714 e1-4.
10. Tsukamoto, H., et al., Epigenetic cell fate regulation of hepatic stellate cells. Hepatol Res, 2011. 41(7): p. 675-82.
11. Zhao, Q., et al., Epigenetic modifications in hepatic stellate cells contribute to liver fibrosis. Tohoku J Exp Med, 2013. 229(1): p. 35-43.

## Claims

1. A model system for liver fibrosis, said system comprising a liver extracellular matrix, and a combination of mammalian liver cells on said matrix, said combination comprising:
*(a)* liver progenitor cells,
*(b)* Kupffer cells, and
*(c)* hepatic stellate cells, wherein said hepatic stellate cells comprise activated hepatic stellate cells and/or myofibroblasts that express EZH2, and
wherein said combination comprises, by number, from 70 to 90 percent liver progenitor cells, from 5 to 20 percent Kupffer cells, and from 5 to 20 percent hepatic stellate cells.

2. The model system of claim 1, wherein said liver extracellular matrix and said combination of mammalian liver cells on said matrix are provided in the form of a spheroid.

3. The model system of claim 1 or claim 2, wherein said liver extracellular matrix comprises a decellularized liver disk.

4. The model system of claim 2 or claim 3, wherein said system is provided in a tissue culture dish, or wherein said system is provided in a modular and/or microfluidic device.

5. The model system of any one of claims 1-4, wherein said liver progenitor cells, Kupffer cells and/or hepatic stellate cells are human cells.

6. The model system of claim 5, wherein the liver extracellular matrix is a non-human mammalian liver extracellular matrix.

7. The model system of any one of claims 1-6, wherein said model system comprises liver structures such as biliary ductal structures and/or clustered hepatoctyes.

8. A method of making the model system of any one of claims 1-7, comprising:
*(a)* providing said liver extracellular matrix, and
*(b)* seeding said liver progenitor cells, Kupffer cells and hepatic stellate cells onto said liver extracellular matrix, then,
*(c)* growing said cells on said matrix *in vitro,* and then,
*(d)* inducing fibrosis of said cells,
to thereby form said model system for liver fibrosis,
wherein said growing is carried out for a time of from one week to three weeks, and wherein said inducing comprises activating said hepatic stellate cells by administering a pro-fibrogenic cytokine or chemical to said model system.

9. A method of screening activity of an agent of interest in modulating liver fibrosis, comprising:
*(a)* providing a model system of any one of claims 1-7,
*(b)* contacting said agent of interest to said model system,
*(c)* measuring fibrosis in the model system, and
*(d)* determining whether the fibrosis is increased or decreased in response to the contacting, to thereby screen the activity of the agent of interest in modulating liver fibrosis.

10. The method of claim 9, wherein the model system is provided in a tissue culture dish, or wherein the model system is provided in a modular and/or microfluidic device.

11. The method of claim 9, wherein the model system is for use in implanting onto or into a liver tissue *in vivo.*

12. The method of any one of claims 9-11, wherein said measuring comprises measuring the activity of EZH2 in the model system.

13. The method of any one of claims 9-11, wherein said measuring comprises optical clearing and analysis.

14. The method of any one of claims 9-13, wherein said agent of interest is an EZH2 inhibitor, an angiotension type 1 (AT1) receptor blocker, halofuginone, a lysyl oxidase or lox-like enzyme inhibitor, an A_{2B} adenosine receptor antagonist, or a monoclinal antibody.

15. The method of any one of claims 9-13, wherein said agent of interest is GSK-126, lostatin, or GS-6624 (simtuzumab).

## Patentansprüche

1. Modellsystem für Leberfibrose, wobei das System eine extrazelluläre Lebermatrix und eine Kombination von Säugetier-Leberzellen auf der Matrix umfasst, wobei die Kombination Folgendes umfasst:
*(a)* Lebervorgängerzellen,
*(b)* Kupffer-Zellen, und
*(c)* hepatische Sternzellen, wobei die hepatischen Sternzellen aktivierte hepatische Sternzellen und/oder Myofibroblasten, die EZH2 exprimieren, umfassen, und
wobei die Kombination, der Anzahl nach, von 70 bis 90 Prozent Lebervorläuferzellen, von 5 bis 20 Prozent Kupffer-Zellen und von 5 bis 20 Prozent hepatische Sternzellen umfasst.

2. Modellsystem nach Anspruch 1, wobei die extrazelluläre Lebermatrix und die Kombination von Säugetier-Leberzellen auf der Matrix in der Form eines Sphäroids bereitgestellt werden.

3. Modellsystem nach Anspruch 1 oder Anspruch 2, wobei die extrazelluläre Lebermatrix eine dezellularisierte Leberscheibe umfasst.

4. Modellsystem nach Anspruch 2 oder Anspruch 3, wobei das System in einer Gewebekulturschale bereitgestellt wird oder wobei das System in einer modularen und/oder einer Mikrofluidikvorrichtung bereitgestellt wird.

5. Modellsystem nach einem der Ansprüche 1 bis 4, wobei die Lebervorläuferzellen, die Kupffer-Zellen und/oder die hepatischen Sternzellen menschliche Zellen sind.

6. Modellsystem nach Anspruch 5, wobei die extrazelluläre Lebermatrix eine extrazelluläre nicht-menschliche Säugetier-Lebermatrix ist.

7. Modellsystem nach einem der Ansprüche 1 bis 6, wobei das Modellsystem Leberstrukturen, wie Gallengangstrukturen und/oder zusammengeballte Hepatozyten, umfasst.

8. Verfahren zum Herstellen des Modellsystems nach einem der Ansprüche 1 bis 7, wobei das Verfahren Folgendes umfasst:
*(a)* das Bereitstellen der extrazellulären Lebermatrix, und
*(b)* das Impfen der Lebervorgängerzellen, der Kupffer-Zellen und der hepatischen Sternzellen auf die extrazelluläre Lebermatrix, danach
*(c)* das Kultivieren der Zellen auf der Matrix *in vitro,* und danach
*(d)* das Induzieren einer Fibrose der Zellen,
um dadurch das Modellsystem für Leberfibrose zu bilden,
wobei das Kultivieren über eine Zeit von einer Woche bis drei Wochen ausgeführt wird und wobei das Induzieren das Aktivieren der hepatischen Sternzellen durch Verabreichen eines pro-fibrogenen Zytokins oder einer Chemikalie an das Modellsystem umfasst.

9. Verfahren zum Prüfen der Aktivität eines Wirkstoffs von Interesse beim Modulieren von Leberfibrose, das Folgendes umfasst:
*(a)* das Bereitstellen eines Modellsystems nach einem der Ansprüche 1 bis 7,
*(b)* das In-Kontakt-Bringen des Wirkstoffs von Interesse mit dem Modellsystem,
*(c)* das Messen der Fibrose in dem Modellsystem, und
*(d)* das Feststellen, ob die Fibrose als Reaktion auf das In-Kontakt-Bringen gesteigert oder vermindert wird, um dadurch die Aktivität des Wirkstoffs von Interesse beim Modulieren von Leberfibrose zu prüfen.

10. Verfahren nach Anspruch 9, wobei das Modellsystem in einer Gewebekulturschale bereitgestellt wird oder wobei das Modellsystem in einer modularen und/oder einer Mikrofluidikvorrichtung bereitgestellt wird.

11. Verfahren nach Anspruch 9, wobei das Modellsystem zur Verwendung beim Implantieren auf oder in ein Lebergewebe *in vivo* dient.

12. Verfahren nach einem der Ansprüche 9 bis 11, wobei das Messen das Messen der Aktivität von EZH2 in dem Modellsystem umfasst.

13. Verfahren nach einem der Ansprüche 9 bis 11, wobei das Messen optische Klärung und Analyse umfasst.

14. Verfahren nach einem der Ansprüche 9 bis 13, wobei der Wirkstoff von Interesse ein EZH2-Inhibitor, ein Angiotensin-Typ-1- (AT1-) Rezeptorblocker, Halofuginon, ein Lysyloxidase- oder LOX-ähnlicher Enzyminhibitor, ein A_{2B}-Adenosin-Rezeptorantagonist oder ein monoklonaler Antikörper ist.

15. Verfahren nach einem der Ansprüche 9 bis 13, wobei der Wirkstoff von Interesse GSK-126, Lostatin oder GS-6624 (Simtuzumab) ist.

## Revendications

1. Système modèle pour une fibrose hépatique, ledit système comprenant une matrice extracellulaire hépatique, et une combinaison de cellules hépatiques de mammifère sur ladite matrice, ladite combinaison comprenant :
*(a)* des cellules progénitrices hépatiques,
*(b)* des cellules de Kupffer, et
*(c)* des cellules stellaires hépatiques, dans lequel lesdites cellules stellaires hépatiques comprennent des cellules stellaires hépatiques activées et/ou des myofibroblastes qui expriment EZH2, et
dans lequel ladite combinaison comprend, en nombre, de 70 à 90 pour cent de cellules progénitrices hépatiques, de 5 à 20 pour cent de cellules de Kupffer, et de 5 à 20 pour cent de cellules stellaires hépatiques.

2. Système modèle selon la revendication 1, dans lequel ladite matrice extracellulaire hépatique et ladite combinaison de cellules hépatiques de mammifère sur ladite matrice sont fournies sous la forme d'un sphéroïde.

3. Système modèle selon la revendication 1 ou la revendication 2, dans lequel ladite matrice extracellulaire hépatique comprend un disque hépatique décelluralisé.

4. Système modèle selon la revendication 2 ou la revendication 3, où ledit système est fourni dans une boîte de culture tissulaire, ou où ledit système est fourni dans un dispositif modulaire et/ou microfluidique.

5. Système modèle selon l'une quelconque des revendications 1 à 4, dans lequel lesdites cellules hépatiques progénitrices, lesdites cellules de Kupffer et/ou lesdites cellules stellaires hépatiques sont des cellules humaines.

6. Système modèle selon la revendication 5, dans lequel la matrice extracellulaire hépatique est une matrice extracellulaire hépatique de mammifère non--humain.

7. Système modèle selon l'une quelconque des revendications 1 à 6, dans lequel ledit système modèle comprend des structures hépatiques telles que des structures des voies biliaires et/ou des hépatocytes groupés.

8. Procédé de fabrication du système modèle selon l'une quelconque des revendications 1 à 7, comprenant les étapes suivantes :
*(a)* fourniture de ladite matrice extracellulaire hépatique ; et
*(b)* ensemencement desdites cellules progénitrices hépatiques, desdites cellules de Kupffer et des cellules stellaires hépatiques sur ladite matrice extracellulaire hépatique ; puis
*(c)* culture desdites cellules sur ladite matrice *in vitro* ; et puis
*(d)* induction d'une fibrose desdites cellules,
pour former ainsi ledit système modèle pour la fibrose hépatique ;
dans lequel ladite culture est effectuée pendant une durée d'une à trois semaines, et dans lequel ladite induction comprend l'activation desdites cellules stellaires hépatiques par administration d'une cytokine pro-fibrinogène ou d'un produit chimique audit système modèle.

9. Procédé de criblage de l'activité d'un agent d'intérêt dans la modulation d'une fibrose hépatique, comprenant les étapes suivantes :
*(a)* fourniture d'un système modèle selon l'une quelconque des revendications 1 à 7 ;
*(b)* mise en contact dudit agent d'intérêt avec ledit système modèle ;
*(c)* mesure de la fibrose dans le système modèle ; et
*(d)* détermination portant sur si la fibrose s'est accrue ou réduite en réponse à la mise en contact, pour cribler ainsi l'activité de l'agent d'intérêt dans la modulation de la fibrose hépatique.

10. Procédé selon la revendication 9, dans lequel le système modèle est fourni dans une boîte de culture tissulaire ou dans lequel le système modèle est fourni dans un dispositif modulaire et/ou microfluidique.

11. Procédé selon la revendication 9, dans lequel le système modèle est destiné à être utilisé pour une implantation sur ou dans un tissu hépatique *in vivo.*

12. Procédé selon l'une quelconque des revendications 9 à 11, dans lequel ladite mesure comprend la mesure de l'activité d'EZH2 dans le système modèle

13. Procédé selon l'une quelconque des revendications 9 à 11, dans lequel ladite mesure comprend un nettoyage et une analyse optiques.

14. Procédé selon l'une quelconque des revendications 9 à 13, dans lequel ledit agent d'intérêt est un inhibiteur d'EZH2, un antagoniste des récepteurs de l'angiotensine de type 1 (AT1), une halofuginone, un inhibiteur de la lysyl-oxidase ou d'enzyme de type lox, un antagoniste des récepteurs de l'adénosine A_{2B}, ou un anticorps monoclinal.

15. Procédé selon l'une quelconque des revendications 9 à 13, dans lequel ledit agent d'intérêt est le GSK-126, la lostatine, ou le GS-6624 (simtuzumab).
